# EUROPEAN PATENT SPECIFICATION

(11) **EP 4 490 140 B1**
(45) Date of publication and mention of the grant of the patent: **18.02.2026**
(21) Application number: 23717261.4
(22) Date of filing: 05.03.2023
(51) Int. Cl.: C07C 67/08, C07C 69/00, C08F 283/01, C08F 290/06, C08F 290/14, C08K 5/00, C08L 67/06, C08L 67/07

(54) **CROSSLINKED POLYESTERS WITH REDUCED FLAMMABILITY**
VERNETZTE POLYESTER MIT VERRINGERTER ENTFLAMMBARKEIT
POLYESTERS RÉTICULÉS À INFLAMMABILITÉ RÉDUITE

(30) Priority: 10.03.2022 US 202263318411 P
(43) Date of publication of application: 15.01.2025
(73) Proprietor: Bromine Compounds Ltd., 8410101 Beer-Sheva (IL)
(72) Inventor: DICHTER, Shay, 8502500 Meitar (IL); ZILBERMAN, Joseph, 3686409 Nesher (IL); BERUBEN, Dov, 8422905 Beer-Sheva (IL); EDEN, Eyal, 6082633 Shoham (IL); LEIFER, Marc, 8060000 Mitzpe Ramon (IL)
(74) Representative: Potter Clarkson
(86) International application number: PCT/IL2023/050222
(87) International publication number: WO 2023/170669

(56) References cited:
- EP-B1- 2 864 117
- WO-A1-2019/130306
- JP-A- S63 215 713
- US-A- 3 763 644
- US-A- 4 367 315

## Description

Unsaturated polyesters are prepared from difunctional monomers, one of which contains a carbon-carbon double bond. That is, in addition to conventional dicarboxylic acids and glycols, there is also an unsaturated comonomer with a reactive C=C bond which takes part in the polyester formation reaction. Because the reactive carbon-carbon double bond is incorporated into the linear polyester backbone, the so-formed unsaturated polyester can be crosslinked by undergoing a subsequent polymerization reaction. For example, an unsaturated polyester is dissolved in a monomer solution such as styrene; in the presence of a free radical initiator, a crosslinking reaction takes place, i.e., styrene crosslinks the polyester chains, affording the cured, thermoset polyester.

Polymers in commercial use contain auxiliary agents to improve their processing and their final properties, for example, flame retardants (FR) to reduce the flammability of the finished product, and unsaturated polyesters are no exception. Flame retardants generally fall into two categories: non-reactive flame retardants, i.e., compounds which are simply added to the polymeric matrix; and reactive flame retardants, i.e., compounds possessing a reactive functional group enabling their incorporation into the polymeric backbone under consideration. The use of reactive flame retardants is more common in unsaturated polyesters and there are in fact two approaches to reduce the flammability of unsaturated polyesters with the aid of reactive flame retardants, specifically, halogenated flame retardants.

One approach is by using a suitable halogenated (brominated) monomer which can take part in the polyester formation reaction, e.g., a brominated diol. One important brominated diol flame retardant which is currently in commercial use is dibromoneopentyl glycol (also named FR-522 and DBNPG), available in a powder form, with high bromine content (~60 wt.%; Bromine added to the polyester accounts for the reduced flammability):

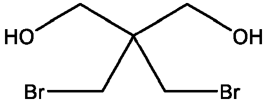

Because it is difunctional, when DBNPG is added during the polymerization reaction of the unsaturated linear polyester, it becomes part of the unsaturated polyester backbone. The performance of DBNPG in unsaturated polyesters is very good. However, the industry seeks replacements, anticipating restrictions on the use of DBNPG. Another example of such an approach - imparting flame retardancy to unsaturated polyesters using a brominated diol - is found in US 4,021,506, where the flame retardant was brominated di(hydroxyalkyl)-benzimidazolone.

The second approach to reducing the flammability of unsaturated polyesters is by addition of a brominated vinyl monomer to the liquid resin formulation, i.e., to the solution consisting of the linear unsaturated polyester dissolved in a monomer solvent, before the crosslinking reaction takes place. A brominated flame retardant which suggests itself is a brominated styrene, e.g., ortho- and para- bromostyrene; see J. Appl. Polym. Sci. 22, 3177 (1978). These brominated styrene derivatives are in a liquid state at room temperature and can be added to a curable solution of unsaturated polyesters in styrene, to create bromostyrenecrosslinked polyester.

A somewhat similar approach was also shown in US 4,367,315. The liquid brominated monomer selected was a brominated ester of acrylic acid of the formula: 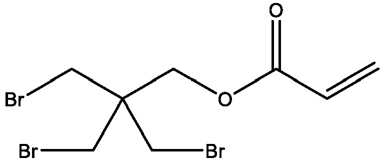 [common chemical names: 3-bromo-2,2-bis(bromomethyl)propyl ester of acrylic acid; 3-bromo-2,2-bis(bromomethyl)-1-propyl acrylate; and trinol acrylate (abbreviated sometimes herein "TA")]. In US 4,367,315, Example I, a polymerization reaction forming an unsaturated polyester is shown. The polyester was recovered in the form of a brittle material, which was then dissolved in styrene (Example II of US 4,367,315), to afford a liquid resin amenable to crosslinking. Next, trinol acrylate was added to the resin solution, alongside a peroxide initiator, and the flame retarded, crosslinked finished product was obtained (Example III of US 4,367,315).

It is of note that trinol acrylate used in Example III of US 4,367,315 was prepared by reacting equimolar amounts of tribromoneopentyl alcohol (also called herein trinol, FR-513 and TBNPA) and acrylic acid:

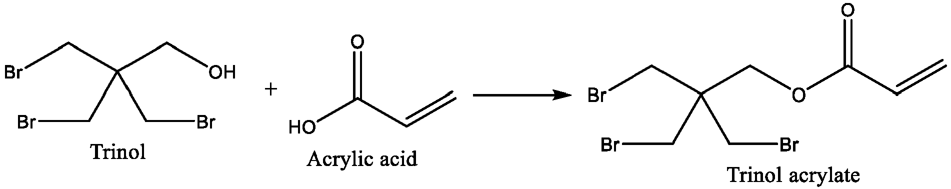

The esterification reaction was not followed by purification of trinol acrylate. In fact, the characteristics of the reaction product reported in Example III of US 4,367,315 indicate that the esterification reaction did not reach completion: an acid number of 12 mg KOH/g and OH number of 34 mg KOH/g are ascribed to the presence of residual acrylic acid and TBNPA. Their presence in the trinol acrylate used in US 4,367,315 is not insignificant, as calculations based on that data show that there are ~1.5 wt.% and ~20.0 wt.% residual acid and trinol, respectively, in the trinol acrylate. This is in line with comparative data given below, showing that a reaction between equimolar amounts of TBNPA and acrylic acid leads to impure (i.e., trinol-containing) product. Stated otherwise, an 80/20 mixture of trinol acrylate/TBNPA was used in US 4,367,315.

Unfortunately, trinol acrylate is not easy to purify. An old patent US 3,480,600 reports that an attempt to purify the monomer by distillation under reduced pressure resulted in thermopolymerization. Another published procedure employing distillation to purify the crude trinol acrylate is found in WO 2007/007332. In WO 2011/045780, the crude product was purified by chromatography; a technique not readily applicable on industrial scale.

For many applications, it can be assumed that a crude product consisting of trinol acrylate and unreacted TBNPA would be acceptable, because TBNPA contributes on its own to the fire-resistance of the polymer matrix, due to its high bromine content.

It is not the case, however, for crosslinked polyesters. We have now studied the effect of unreacted TBNPA present in trinol acrylate on the performance of trinol acrylate in crosslinked polyesters and found that flame-resistance, mechanical and optical properties of crosslinked polyesters can be improved by minimizing the amount of unreacted TBNPA. That is, an essentially TBNPA-free trinol acrylate performs better than the 80/20 mixture of trinol acrylate/TBNPA, that was used in US 4,367,315.

One aspect of the invention is therefore a method of improving the fire-resistance of a crosslinked polyester, comprising adding an essentially TBNPA-free trinol (meth)acrylate to a solution of unsaturated polyester in a monomer solvent and initiating crosslinking, e.g., with a free radical initiator and optionally an accelerator, to crosslink the polyester (by copolymerizing the polyester, the monomer solvent and trinol acrylate).

Another aspect of the invention is a composition comprising an unsaturated polyester dissolved in a monomer solution, wherein the composition comprises an essentially TBNPA-free trinol (meth)acrylate. Ancillary components(s) (e.g., an accelerator) needed to advance the crosslinking reaction may be formulated beforehand with the unsaturated polyester resin in the monomer solution, or added to the liquid resin shortly before use, i.e., by the manufacturer of the finished, cured product, together with a free radical initiator, as described below.

By "an essentially TBNPA-free trinol (meth)acrylate" it is meant that the weight percentage of TBNPA in the trinol (meth)acrylate product is not more than 5.0 wt.%, less than 2.5 wt.%, less than 1.0 wt.%. By "TBNPA-free trinol (meth)acrylate" we mean that the weight percentage of TBNPA in the trinol acrylate product is less than 0.5 wt.%, less than 0.3 wt.% and even less than 0.1 wt.%, measured by quantitative HPLC analysis. By "curable composition" it is meant that the composition is capable of being transformed from its uncured liquid state to its cured solid state, when exposed to suitable conditions.

By trinol (meth)acrylate, we mean either trinol acrylate or trinol methacrylate, that is, each of the compounds depicted below:

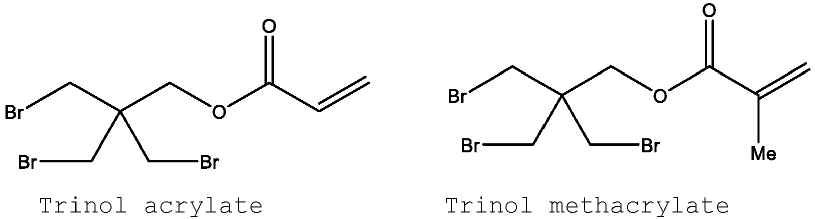

An unsaturated polyester resin for use in the invention is the polycondensation product of:
A) at least one difunctional monomer possessing a C=C double bond that is capable of undergoing a crosslinking reaction, namely, unsaturated dicarboxylic acid and unsaturated anhydrides (for example, maleic anhydride, fumaric acid and fumaric anhydride; other examples include itaconic and citraconic acids) . Unsaturation can also be introduced by dicyclopentadiene maleate or the reaction product of cyclopentadiene and maleic anhydride.
B) at least one non-crosslinkable dicarboxylic acid or dicarboxylic acid anhydride, i.e., aromatic (ortho-phthalic acid, phthalic anhydride, terephthalic acid, isophthalic acid) and aliphatic (HOOC-(CH₂)ₘ-COOH, m≥2, such as succinic acid, glutaric acid, adipic acid, pimelic acid, suberic acid, azelaic acid and sebacic acid), and halogenated derivatives thereof; and
C) at least one diol, for example, glycols such as ethylene glycol, diethylene glycol, propylene glycol, dipropylene glycol, butylene glycol and the like.

In the industry, crosslinked polyesters are commonly called "unsaturated polyesters". However, we use the term "unsaturated polyester" to indicate the uncured, unsaturated polyester resin, whereas the term "crosslinked polymer" indicates the cured, thermoset polymer obtained after the unsaturated linear polyester chains were crosslinked.

Unsaturated polyesters for use in the invention are commercially available in the form of their liquid uncured state, consisting of a clear solution of the resin in the monomer solvent. For example, from DSM Composite Resins, e.g., unsaturated polyester based on ortho-phthalic acid and glycols, dissolved in styrene. Liquid resins can also be prepared by known methods, e.g., solvent-free synthesis, wherein components A), B) and C) are combined in a reaction vessel, the temperature is raised to about 150°C-250°C under nitrogen atmosphere, water formed due the condensation is removed, to obtain a clear melt which is dissolved in the monomer solution, e.g., in styrene, as described in US 4,021,506. The molar ratio (A): (B) is usually from 3:1 to 1:2, e.g., from 3:1 to 1:1. The molar ratio [(A)+(B)] to (C) can be around 1:1, but a slight molar excess of (C) is usually employed, e.g., up to 25% molar excess of (C).

Although styrene is by far the most commonly used solvent monomer, and is often mentioned herein by way of example, the invention is not limited to solutions of the polyester resin in styrene. The polyester resin can be dissolved in, and crosslinked with, other unsaturated organic solvents, i.e., liquid vinyl monomers such as dichlorostyrene, vinyl toluene, vinyl acetate, methyl methacrylate and allyl ethers and difunctional vinyl monomers.

The weight ratio between the unsaturated polyester resin and the monomer solvent can be from 3:1 to about 1:1, e.g., the concentration of the monomer solvent such as styrene in the composition of the invention is from 30 to 60 wt.%, usually up to 50 wt.%. For example, solutions of polyester resins based on ortho-phthalic acid, glycols and maleic anhydride or fumaric acid in styrene which exhibit medium viscosity, e.g., from 900 to 1200 mPa·s (measured at room temperature or slightly above, up to 40°C) and an acid number of less than 100, e.g., from 10 to 50 (the amount of KOH in mg which is required to neutralize the free carboxyl groups contained in 1 g of polyester). Other properties worth mentioning relate to the cured forms of such commercial resins that can be used in the invention, e.g., tensile strength (TS) above 50 Mpa, e.g., TS>70 Mpa or TS> 80 Mpa; and glass transition temperature (Tg) of above 120°C.

Next, an essentially TBNPA-free trinol acrylate is added to the solution of the polyester resin in the monomer solvent, to form the curable composition of the invention. The amount of trinol acrylate used is in the range from 20 to 50 parts by weight (e.g., from 25 to 45 parts, or from 30 to 40 parts) per 100 parts of resin solution [(phr); phr is calculated based on the total of the polyester and monomer solvent]. Such amounts of trinol acrylate supply from ~13 to 33 phr of bromine, e.g., from 16 to 30 phr, or 18 to 25 phr of bromine.

A low level of TBNPA in trinol acrylate is a key requirement of the invention. Owing to the use of high purity trinol acrylate, prepared by the process described below, we can supply effective amounts of bromine to the curable polyester resin composition, to incorporate bromine into the crosslinked polyester but at the same time, obtain a very low concentration of the undesired TBNPA in the curable composition (and in the cured product), e.g., less than 1.5 wt.%, less than 1.0 wt.%, less than 0.3 wt.%, <0.2 wt.%, <0.1 wt.%, and even below 0.05 wt.%, based on the total weight of the curable (liquid) and cured (solid) compositions.

As previously explained, efficient purification of crude trinol acrylate to remove residual, unreacted TBNPA, which was found to interfere with the action of trinol acrylate in unsaturated polyesters, is difficult to achieve. Experimental work reported below shows that trinol (meth)acrylate with a desirable purity level (in terms of residual TBNPA) is attainable through a two-step process, comprising two chemical steps, consisting of the main esterification step and a subsequent esterification reaction intended to eliminate TBNPA:
Step 1: reaction of TBNPA with (meth)acrylic acid: 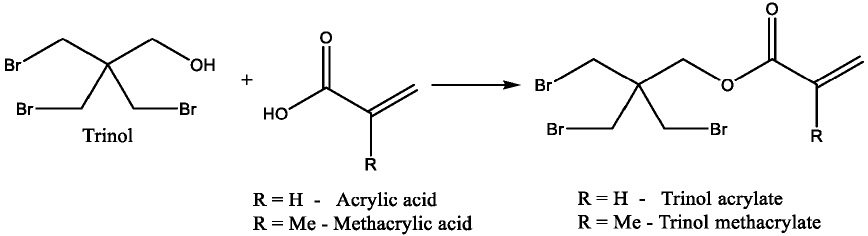 at a molar excess of the acid, e.g., at least 5%, or at least 10% molar excess, e.g., from 20 to 50% molar excess, to form a reaction mixture comprising trinol (meth)acrylate and residual TBNPA; and
Step 2: adding to the reaction mixture a scavenger reactive towards an alcohol to eliminate residual TBNPA, e.g., the scavenger is an esterification agent that has stronger reactivity towards an alcohol than that of (meth)acrylic acid. In step 1, the reaction takes place in a reaction vessel equipped with an azeotropic distillation device to remove water - the byproduct of the esterification reaction. The reactants are refluxed in a suitable organic solvent in the presence of a common esterification catalyst such as concentrated sulfuric acid and a polymerization inhibitor for acrylic monomers, most suitably hydroquinone or its methyl ether derivative, namely, p-methoxyphenol. Air (or oxygen) is bubbled through the reaction mixture along the entire reaction time. Air is necessary for activating the inhibitor para-methoxyphenol. The para-methoxyphenol-oxygen adduct acts as a very efficient polymerization inhibitor. For example, (meth) acrylic acid is added to the reaction vessel that was previously charged with the organic solvent (e.g., an aromatic hydrocarbon such as alkyl-substituted benzene, e.g., toluene) and TBNPA (and the esterification catalyst and polymerization inhibitor); prior to the addition of the (meth)acrylic acid, the reaction mixture is heated to dissolve TBNPA in the solvent. After the addition of (meth)acrylic acid in excess, the reaction temperature is raised to reflux temperature, and water is removed through azeotropic distillation while the organic solvent is condensed and returned to the reaction vessel. Reaction time is usually from 3 to 5 hours.

In step 2, the reaction mixture, consisting mostly of trinol (meth)acrylate dissolved in the organic solvent (e.g., toluene), is cooled to <110°C, and an esterification agent whose reactivity towards an alcohol is stronger than that of (meth)acrylic acid is added to the reaction mass. Suitable esterification agents that act as scavengers are structurally related to acrylic acid, and include reactive derivatives of acrylic acid or methacrylic acid, namely, anhydrides and acyl halides, which can efficiently eliminate the unreacted alcohol (TBNPA) through an esterification reaction, e.g., (meth)acrylic anhydride and acryloyl chloride:

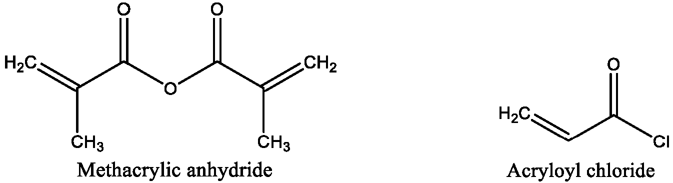

The scavenger, namely, the esterification agent is added in equimolar amount, or in a slight molar excess, relative to the residual TBNPA. The esterification reaction to remove TBNPA takes place under heating, e.g., at above 40°C, say, from 60 to 105 °C. In case of using anhydrides such as for example methacrylic anhydride, the reaction is carried out in the presence of a nitrogen-containing organic base, namely, an amine, e.g., tertiary amines including amino-substituted pyridine derivatives, which are capable of catalyzing an esterification reaction with anhydrides, for example, 4-dimethylbutylamine, 4-dimethylaminopyridine and 4-pyrrolidinopyridine. The amount of the organic base used to catalyze the esterification reaction is from 0.1 to 1 mol per one mol of anhydride. In case of using acyl halides such as for example acryloyl chloride, no additional reagents are required.

Upon completion of the reaction, the reaction mixture is cooled to room temperature and is worked-up to recover an essentially pure trinol (meth)acrylate, through a sequence of aqueous washings and lastly, distillation of the organic solvent (e.g., toluene) at reduced pressure. The reaction mixture is first washed with water, to remove sulfuric acid (the catalyst of step 1) and part of the unreacted acrylic acid (also from step 1). The reaction mixture is then separated into organic and aqueous phases. The organic phase is treated with an aqueous inorganic base, usually bicarbonate, to remove unreacted organic acids, namely, acrylic and methacrylic acids. Next, the inorganic base and sodium salts of the organic acids are washed out with water. Upon phase separation, the organic phase is collected, from which the organic solvent is separated by distillation under reduced pressure to afford the product in the form of a clear yellow liquid.

By the two-step process described above, the concentration of TBNPA in the final trinol (meth)acrylate product can be lowered to 0.1 wt.% and even less, according to quantitative HPLC analysis.

Accordingly, another aspect of the invention is a process for preparing an essentially TBNPA-free trinol (meth)acrylate, comprising reacting (meth)acrylic acid with TBNPA: 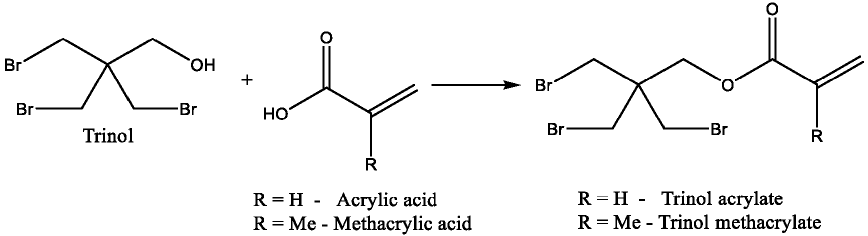 at a molar excess of the acid, to form a reaction mixture comprising trinol (meth)acrylate and residual TBNPA; adding to the reaction mixture a scavenger reactive towards an alcohol to eliminate residual TBNPA and recovering essentially TBNPA-free trinol (meth)acrylate.

The essentially TBNPA-free trinol (meth)acrylate obtained by the two-step process described above, e.g., trinol acrylate (which forms another aspect of the invention) is added to the resin/monomer solution. At room temperature, trinol acrylate is a low-viscosity liquid which mixes readily with the solution of unsaturated polyester resin in the solvent monomer, forming the curable composition of the invention as a clear, homogeneous, easily processable mixture. It is of note that brominated flame retardants available on the marketplace are mostly solid compounds, and their dissolution in the monomer solvent (i.e., styrene) makes the curable mixture difficult to process. For example, we have tested a brominated epoxy polymeric flame retardant (F-3014) and also the abovementioned FR-522 (in the system under consideration, they both act as additive flame retardants) and observed difficulties during processing and loss of transparency in the finished product.

The curing process, i.e., the copolymerization of the unsaturated polyester, the solvent monomer and trinol acrylate flame retardant is advanced with the aid of an accelerator and an initiator. As pointed out above, the accelerator can be formulated beforehand with the resin in the monomer solution (in absence of the initiator, the accelerator cannot cause premature curing) or added together with the initiator just before the resin is cured.

Cobalt salts of organic acids are useful as accelerators, e.g., cobalt bis(2-ethylhexanoate), cobalt naphthenate and cobalt neodecanoate. The cobalt salts are available in the form of solutions with 1 to 10 wt.%. e.g., ~ 6 wt.% Co²⁺. In general, the amount of accelerator added is usually from 0.001 to 0.05 phr, e.g., 0.001 to 0.005 phr, or 0.001 to 0.002 phr, or 0.001 to 0.015 phr (parts per hundred resin as a metal content).

The initiator is a free radical initiator, for example, of the peroxide or hydroperoxide type, usually an organic peroxide such as aliphatic peroxide (methyl ethyl ketone peroxide, acetyl peroxide, chlorinated acyl peroxide, lauryl peroxide and stearyl peroxide), aromatic peroxides (such as benzoyl peroxide, dichlorobenzoyl peroxide) and peresters (such as tertbutyl peroxybenzoate). In general, an acceptable amount of the initiator is in the range from 0.5 to 1.5% by weight, calculated based on the polyester resin or polyester/monomer solution. The entire amount of the initiator can be either added at the beginning of the copolymerization reaction or divided into portions supplied as the reaction progresses.

The concentrations of the accelerator (promoter) and initiator are carefully adjusted, to control the rate of gelation and achieve full curing of the resin. The lower the concentrations used, the higher the pot life. In some applications very high pot life is needed, and therefore the cobalt accelerator is used at a concentration of less than 1%, less than 0.5%, less than 0.1% by weight.

The composition is thoroughly stirred and after a brief treatment under vacuum, the homogeneous, clear mixture is cured in a suitable mold, e.g., at a temperature in the range from 70 to 100°C, for at least 5-12 hours. Usually the post-vacuum liquid composition is transferred to an oven for curing after it has stayed at room temperature for a while to form a gel; then the gel material is placed in an oven.

Other additives, e.g., pigments (up to about 3.0 phr) and reinforcing fillers (from 20 to 120 phr, e.g., glass fibers and mineral powder) may also be added to the composition of the invention before the initiator is added, to color the resin and impart other useful properties. For example, dispersing agents (such as DISPERBYK^{®}-192) or BYK-A-55 (a silicone-free polymerbased air release additive) can be added to improve heat distribution in the hardened thermoset.

The composition of the invention is suitable for all typical uses of crosslinked polyesters. It may be combined with a reinforcement material (e.g. glass fibers) to create the corresponding composite (e.g., glass reinforced polyester), e.g., by lamination. Typical uses of the trinol acrylatecontaining polyester resins provided by the invention include construction, marine accessories, boat hulls, automobile parts, pipes, tanks, closure and body panels. Unsaturated polyester resins also find uses in coatings.

The experimental results reported below indicate that the cured polyester that is flame retarded with trinol acrylate shows reduced flammability and possesses good mechanical properties.

Accordingly, the invention further relates to a trinol acrylate-crosslinked polyester, with less than 0.3 wt.% of TBNPA, based on the total weight of the crosslinked polyester.

That is, the invention provides a trinol acrylate-crosslinked polyester, in which polyester chains are crosslinked to form a network, e.g., as schematically shown below: 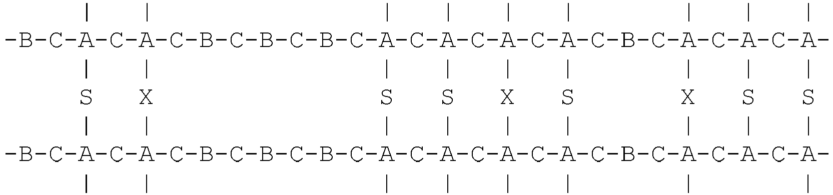
wherein A, B and C are the structural units corresponding to the monomers A, B, and C that form the polyester backbone chain; as previously defined (A - the unsaturated diacid/anhydride; B - the non-crosslinkable diacid/anhydride; and C - glycol), with A and B being randomly distributed across the polyester backbone; S is a structural unit corresponding to a styrene monomer; and X is a structural unit corresponding to a trinol acrylate monomer;
wherein the bromine content of the crosslinked polyester is from 14 to 18 percent by weight, e.g., from 15 to 18 percent by weight, characterized in that there are no bromine atoms alongside the polyester backbone chain, bromine atoms being exclusively incorporated in the X units joining two polyester chains together, and further characterized in that the concentration of non-copolymerized brominated compounds such as TBNPA is less than 1.5 wt.%, less than 1.0 wt.%, less than 0.3 wt.%, <0.2 wt.%, <0.1 wt.%, and even below 0.05 wt.% (e.g., from 0.02 to 0.03 wt%), calculated based on the total weight of the crosslinked polyester (absent filler component).

Analytical methods to determine and quantify TBNPA in the liquid, unsaturated polyester solution (in a monomer solvent, e.g., in styrene), or in the solid, thermosetting crosslinked polyester, include gas chromatography. When determined in the crosslinked polyester, TBNPA could be leached out using a well-known Soxhlet extraction method, followed by analysis of the extract by gas chromatography.

### Examples

### Materials

**Table 1**

| Component (manufacturer) | General description | Function |
|---|---|---|
| Palatal^{®} P-6-01 (DSM Composite Resins) | An unsaturated polyester based on ortho-phthalic acid and standard glycols dissolved in styrene | Resin/matrix |
| FR-513 (ICL Industrial Products) | Tribromoneopentyl alcohol (Trinol, TBNPA) | Brominated FR |
| Trinol Acrylate (Preparations 1 and 2) | Brominated acrylate monomer | Brominated FR |
| FR-522 (ICL Industrial Products) | Dibromoneopentyl glycol (Dinol, DBNPG) | Brominated FR |
| F-3014 (ICL Industrial Products) | End capped Brominated Epoxy | Brominated FR |
| NL-51P (Akzo Nobel Functional Chemicals) | Cobalt bis(2-ethylhexanoate), 35%, in 2,2,4-Trimethyl-1,3-pentanediol diisobutanoate (TXIB), 40%, and heavy hydrotreated naphtha (petroleum), 25% (6% of cobalt content) | Accelerator |
| Andonox KP-9 (Norac Andos) | Methyl ethyl ketone peroxide (MEKP), 30-40% solution, in dimethyl phthalate, 40-50% and proprietary phlegmatiser, 10-20%. | Initiator |

### Methods

1)GC analyses of the reaction mixtures and the final TA products were conducted on an Agilent 8860 II apparatus with a capillary column (Agilent 19091-J-413 HP-5 30 m). A quantitative HPLC analysis of residual tribromoneopentyl alcohol in trinol acrylate was performed employing an Agilent Technology HPLC 1200 series instrument fitted with a UV detector. A Super Phenyl Hexyl ultracore column, 150*4.6 mm, 2.5 µm column was used.
2) Flammability: the flammability test was carried out according to the UL-94-HB standard, applying the horizontal burning test on specimens of 3.2 mm thickness. A flammability hood was used as recommended by UL-94V.
3) 3-Point bending properties

A three-point bending test was carried out according to the ASTM D-790 method with V = 1.3 mm/min on a Zwick Z-010 material testing machine.

### Preparation 1 (of the invention) Preparation of high purity trinol acrylate

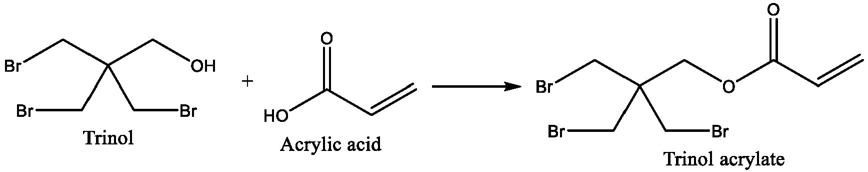

### Step 1: reaction of acrylic acid/ tribromoneopentyl alcohol at 1.25 molar ratio

A 1-liter reactor, equipped with a mechanical stirrer, a thermometer, a Dean-Stark trap combined with a reflux condenser and a narrow glass tube for bubbling air was charged with 320 ml toluene, 400 g (1.23 mol) tribromoneopentyl alcohol, 6 g 96% H₂SO₄ and 4 g para-methoxyphenol. The tribromoneopentyl alcohol slurry was heated up and at about 80°C it turned into a clear yellow solution. Then 110.8 g (1.54 mol) acrylic acid was added, and the reactor contents were heated to reflux. The water formed was removed as an azeotrope with toluene at ~117-127°C over a period of 3 h. Analysis showed 1.7% residual tribromoneopentyl alcohol (GC area %) and 6.7% unreacted excess acrylic acid.

### Step 2: elimination of residual TBNPA.

After cooling the reactor contents to 80°C, methacrylic anhydride (10.8 g, 0.07 mol) and 4-dimethylaminopyridine (DMAP, 3.6 g) were added. The reaction mixture was heated at 80°C for 1 h. The final reaction mixture was washed at ambient temperature with 320 g water to eliminate the sulfuric acid and part of the acrylic acid. After phase separation the acidic organic phase was washed with 320 g aq. 7% NaHCO₃ to remove acrylic and methacrylic acids. Another 320 g water wash removed residual bicarbonate, sodium acrylate and methacrylate. Toluene was stripped under vacuum affording the final product in the form of a clear yellow liquid, 456 g, yield 97.8% based on tribromoneopentyl alcohol. The content of tribromoneopentyl alcohol in the final product was below 0.1% wt. according to quantitative HPLC analysis.

### Preparation 2 (of the invention)

### Preparation of high purity trinol acrylate

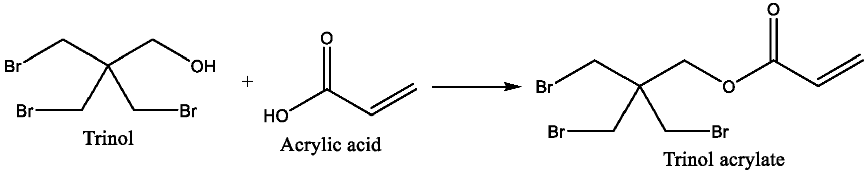

### Step 1:

The same as in Preparation 1.

### Step 2: Elimination of residual TBNPA.

Acryloyl chloride was added (0.03 mol, 2.7 g) and the reaction mixture was stirred at 60°C for 1 hr. Washings and toluene stripping were the same as in Preparation 1, step 2.

The content of tribromoneopentyl alcohol in the final product was below 0.1% wt. according to quantitative HPLC analysis.

### Preparation 3 (comparative)

### Preparation of trinol acrylate using acrylic acid/ tribromoneopentyl alcohol molar ratio of 1:1 (according to US 4,367,315)

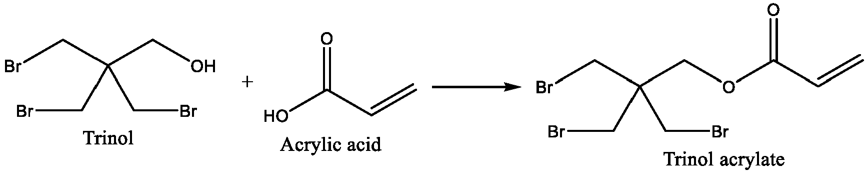

A 0.1-liter reactor, equipped with a mechanical stirrer, a thermometer, a Dean-Stark trap combined with a reflux condenser was charged with 30 ml toluene, 32.5 g (0.1 mol) tribromoneopentyl alcohol, 0.5 g 96% H₂SO₄ and 0.3 g para-methoxyphenol. The tribromoneopentyl alcohol slurry was heated up and at about 80°C it turned into a clear yellow solution. Then 7.2 g (0.1 mol) acrylic acid was added, and the reactor contents were heated to reflux. The water formed was removed as an azeotrope with toluene at ~117-136°C over a period of 3 h. Analysis showed 11.1% residual tribromoneopentyl alcohol (GC area %) and only traces of acrylic acid. The conversion of tribromoneopentyl alcohol into its acrylate cannot proceed further without the presence of acrylic acid.

### Examples 1-2 (comparative) and 3-5 (of the invention)

### Crosslinking of unsaturated polyester in styrene solution in the presence of trinol acrylate and different amounts of TBNPA

The goal of the study was to evaluate the effect of "residual" TBNPA in trinol acrylate on the performance of the latter as a flame retardant in crosslinked unsaturated polyesters as well as its influence on mechanical and physical properties. To this end, the polyester was crosslinked in a styrene monomer solution, in the presence of trinol acrylate and varied concentrations of TBNPA [i.e., (1) ultra-pure trinol acrylate, (2) 95:5 by weight and (3) 80:20 by weight mixtures of trinol acrylate/ TBNPA] and the flammability and mechanical properties of crosslinked unsaturated polyester were determined.

Unsaturated polyester resin Palatal^{®} P-6-01, consisting of unsaturated polyester dissolved in styrene, was used as a reference (Example 1 for neat resin). Example 2 was designed to match the conditions of Example III in US 4,367,315. In Examples 3, 4 and 5, highly pure and ultra-pure trinol acrylate grades were used. In Examples 2 to 4, the calculated content of Bromine was 15% by weight. In Example 5, bromine content was 18% by weight.

Based on the desired batch scale, the required quantity of NL-51P (Cobalt, 6%) was added to the Palatal^{®} P-6-01 base resin to obtain accelerated base resin. Trinol acrylate, or a suitably proportioned mixture of trinol acrylate/TBNPA, was added to the solution followed by Andonox KP-9 (1.25 wt.% of MEKP). The mixture was stirred for at least 90 seconds, and the sample was inserted into a vacuum chamber for at least 5 minutes. The specimen was cast in a silicon rubber mold and the mold was dried in an oven for 12 hours at 80 °C. The resulting specimens were conditioned for a week at 23 °C. The compositions of the samples of Examples 1-5 and the results of a flammability test and mechanical tests are set out in Table 2. The burning rate in Example 1 was 16.7 mm/min.

**Table 2**

| | Example 1 reference | Example 2 (80:20) TA/TBNPA | Example 3 (95:5) TA/TBNPA | Example 4 ~100 (ultra-pure TA) invention | Example 5 ~100 (ultra-pure TA) invention |
|---|---|---|---|---|---|
| | | US 4367315 | invention | | |
| **Composition (wt.%)** | | | | | |
| Resin (palatal) | 100 | 77.0 | 76.5 | 76.3 | 71.6 |
| Trinol acrylate | | 18.4 | 22.3 | 23.7 | 28.4 |
| TBNPA | | 4.6 | 1.2 | <0.05 | <0.05 |
| Bromine (calculated) | | 15 | 15 | 15 | 18 |

| **Flammability test (UL-94-HB/3.2mm test)** | | | | | |
|---|---|---|---|---|---|
| Total burning time (s) | 04:29 | 00:20 | 00:16 | 00:13 | 0: 09 |
| Samples reached 25mm line | yes | no | no | no | no |
| Samples reached 100mm line | yes | no | no | no | no |

| **Mechanical properties (3-point bending test)** | | | | | |
|---|---|---|---|---|---|
| max. stress (MPa) [SD] | 54.3[8.7] | 88.3[5.5] | 94.9[3.0] | 99.2 [3.3] | 109.8 [7.5] |
| max. strain (%) [SD] | 1.7[0.3] | 3.5 [0.4] | 3.4[0.1] | 4.4[0.2] | 4.2[0.7] |
| Modulus (MPa) [SD] | 3121[67] | 2279 (219) | 2993[56] | 2539 [251] | 2808 [206] |

The results show that the burning time of the sample decreases with decreasing trinol content. As to the mechanical properties, it is seen that compared to the neat, brittle resin of Example 1, the incorporation of trinol acrylate into the crosslinked polyester offers a useful combination of reduced module, increased strain alongside high stress, with the trend improving in Examples 3 to 5 (of the invention) versus Example 2, that is, mechanical properties are improved with TBNPA content in the range ≤5% by weight.

### Examples 6-8 (all comparative)

### Crosslinking of unsaturated polyester in styrene solution in the presence of non-reactive flame retardants

In this set of examples, Palatal^{®} P-6-01 was crosslinked in the absence of any flame retardant (Example 6) and in the presence of non-reactive flame retardants: in Example 7, DBNPG (FR-522) was tested and in Example 8, end capped Brominated Epoxy F-3014 was tested. Both FR-522 and F-3014 are solid flame retardants.

Based on the desired batch scale, the required quantity of NL-51P (Cobalt, 6%) was added to the base Palatal^{®} P-6-01 resin to obtain accelerated based resin as a liquid substance. The calculated amount of solid flame retardant (FR-522 or F-3014) was added and the resulting mixture was placed in an oven at 150 °C for 3 hours to melt/dissolve the flame retardant. The obtained homogenous liquid was cooled to room temperature. In both Examples 7 and 8, highly viscous liquids were formed due to the addition of the solid flame retardant. While the flame retardantfree sample (Example 6) was found to be suitable for further processing to accomplish the crosslinking reaction, the viscous liquids of Examples 7 and 8 had to be heated to 50-60 °C before the addition of the crosslinking agent.

For the crosslinking step, a calculated amount of Andonox KP-9 (1.25 wt.% of methyl ethyl ketone peroxide (MEKP)) was added and the mixture was stirred for at least 90 seconds. The sample was inserted into a vacuum chamber for at least 5 minutes and the specimen was cast in a silicon rubber mold. The mold was dried in an oven for 12 hours at 80 °C. The resulting specimens were conditioned for a week at 23 °C and tested. The composition and the results are presented in Table 3. The burning rate in Example 7 was 7.5 mm/min. In Table 3, the quantity of the charged materials is expressed in parts per hundred resin (phr) which is the quantity of the unit of additives per 100 units of the base polymer (Palatal^{®} P-6-01). The accelerator NL-51P and the initiator Andonox KP-9 were used in 0.001 phr and 0.015 phr, respectively.

**Table 3**

| Ex. | FR-522 phr | F-3014 phr | UL-94-HB | | | Remarks |
|---|---|---|---|---|---|---|
| | | | Total burning time, sec | Sample reach 25 mm line | Sample reach 100 mm line | |
| 6 | | | 07:02 | Yes | Yes | Good processability of the pre-cured liquid resin |
| 7 | 25 | | 01:38 | No | No | Melting to dissolve FR; on cooling, high viscosity, sticky sample was obtained, which was heated to 50-60 °C before addition of crosslinking agent |
| 8 | | 25 | 01:32 | 1 of 4 | No | Melting to dissolve FR; on cooling, high viscosity sample was obtained, which was heated to 50-60 °C before addition of crosslinking agent |

The results indicate the difficulties encountered in formulating solid brominated flame retardants into solutions of liquid unsaturated polyesters. Furthermore, the procedure of Examples 7 and 8 resulted in a loss of transparency, i.e., creation of opaque test specimens.

## Claims

1. A composition comprising an unsaturated polyester dissolved in a monomer solution together with trinol (meth)acrylate, wherein the composition is essentially free of tribromoneopentyl alcohol (TBNPA) of the formula:

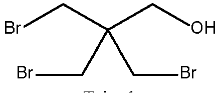

2. A composition according to claim 1, comprising from 25 to 45 parts of trinol acrylate per hundred parts of resin (phr), wherein trinol acrylate has the formula:

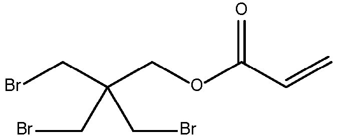

3. A composition according to claim 1 or 2, wherein the concentration of TBNPA is less than 0.3 wt.%, calculated based on the total weight of the composition.

4. A composition according to the preceding claims, comprising an accelerator.

5. A composition according to claim 4, comprising a free radical initiator.

6. A method of improving the fire-resistance of a crosslinked polyester, comprising adding an essentially TBNPA-free trinol (meth)acrylate, accelerator and a free radical initiator to a solution of unsaturated polyester in a monomer solvent, thereby forming a curable composition, and initiating crosslinking, to crosslink the polyester.

7. A method according to claim 6, wherein the concentration of TBNPA in the curable composition is less than 0.3 wt.%, based on the total weight of the composition.

8. Crosslinked polyester obtainable by the methods of claims 6 or 7.

9. A trinol (meth)acrylate-crosslinked polyester, with less than 0.3 wt.%. of TBNPA, based on the total weight of the crosslinked polyester.

10. A trinol (meth)acrylate-crosslinked polyester according to claim 9, wherein the bromine content of the crosslinked polyester is from 14 to 18 percent by weight, with no bromine atoms alongside the polyester backbone chain, bromine atoms being exclusively incorporated in the structural unit corresponding to the trinol a(meth)acrylate that join two polyester chains together, wherein the concentration of non-copolymerized TBNPA is less than 0.1 wt.%, calculated based on the total weight of the crosslinked polyester.

11. A process for preparing an essentially TBNPA-free trinol (meth)acrylate, comprising reacting (meth)acrylic acid with TBNPA: 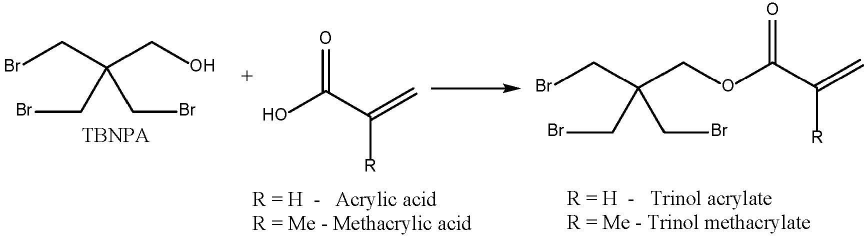 at a molar excess of the acid, to form a reaction mixture comprising trinol (meth)acrylate and residual TBNPA; adding to the reaction mixture a scavenger reactive towards an alcohol to eliminate residual TBNPA and recovering essentially TBNPA-free trinol (meth)acrylate.

12. A process according to claim 11, wherein the reaction of (meth)acrylic acid and TBNPA takes place in an organic solvent in the presence of an esterification catalyst and a polymerization inhibitor at reflux temperature, water formed is removed through azeotropic distillation while the organic solvent is condensed and returned to the reaction vessel.

13. A process according to claim 11, wherein the scavenger added is an esterification agent whose reactivity towards an alcohol is stronger than that of (meth) acrylic acid, the scavenger being selected from reactive derivatives of (meth)acrylic acid.

14. A process according to claim 12, wherein the esterification agent is (meth)acrylic anhydride or acryloyl chloride:

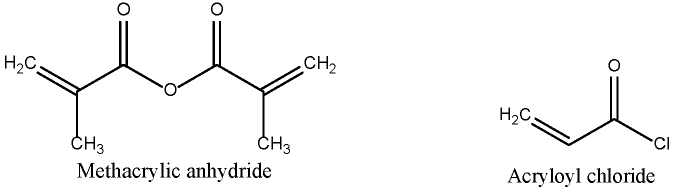

15. The process according to claim 14, wherein the esterification reaction to remove TBNPA takes place under heating, using methacrylic anhydride in the presence of a nitrogen-containing organic base to catalyze the esterification reaction of TBNPA and methacrylic anhydride.

## Patentansprüche

1. Zusammensetzung, umfassend einen in einer Monomerlösung gelösten ungesättigten Polyester zusammen mit Trinol (meth) acrylat, wobei die Zusammensetzung im Wesentlichen frei von Tribromonopentylalkohol (TBNPA) der folgenden Formel ist:

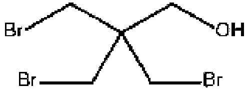

2. Zusammensetzung nach Anspruch 1, umfassend 25 bis 45 Teile Trinolacrylat pro hundert Teile Harz (phr), wobei das Trinolacrylat die folgende Formel aufweist:

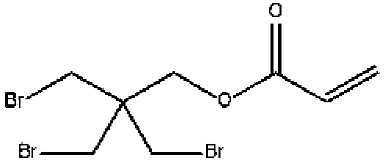

3. Zusammensetzung nach Anspruch 1 oder 2, wobei die Konzentration von TBNPA weniger als 0,3 Gew.-% beträgt, berechnet basierend auf dem Gesamtgewicht der Zusammensetzung.

4. Zusammensetzung nach den vorstehenden Ansprüchen, umfassend einen Beschleuniger.

5. Zusammensetzung nach Anspruch 4, umfassend einen freien Radikalinitiator.

6. Verfahren zur Verbesserung der Feuerbeständigkeit eines vernetzten Polyesters, umfassend die Zugabe eines im Wesentlichen TBNPA-freien Trinol(meth)acrylats, eines Beschleunigers und eines Radikalinitiators zu einer Lösung eines ungesättigten Polyesters in einem Monomerlösungsmittel, wodurch eine härtbare Zusammensetzung gebildet wird, und die Einleitung der Vernetzung, um den Polyester zu vernetzen.

7. Verfahren nach Anspruch 6, wobei die Konzentration von TBNPA in der härtbaren Zusammensetzung weniger als 0,3 Gew.-%, bezogen auf das Gesamtgewicht der Zusammensetzung, beträgt.

8. Vernetzter Polyester, erhältlich nach den Verfahren nach Anspruch 6 oder 7.

9. Trinol(meth)acrylat-vernetzter Polyester, mit weniger als 0,3 Gew.-% TBNPA, basierend auf dem Gesamtgewicht des vernetzten Polyesters.

10. Trinol(meth)acrylat-vernetzter Polyester nach Anspruch 9, wobei der Bromgehalt des vernetzten Polyesters 14 bis 18 Gewichtsprozent beträgt, ohne Bromatome entlang der Polyesterhauptkette, wobei Bromatome ausschließlich in die Struktureinheit eingebaut sind, die dem Trinol(meth)acrylat entspricht, das zwei Polyesterketten miteinander verbindet, wobei die Konzentration an nicht-polymerisiertem TBNPA weniger als 0,1 Gew.-% beträgt, berechnet basierend auf dem Gesamtgewicht des vernetzten Polyesters.

11. Prozess zur Herstellung eines im Wesentlichen TBNPA-freien Trinol(meth)acrylats, umfassend die Umsetzung von (Meth)acrylsäure mit TBNPA: 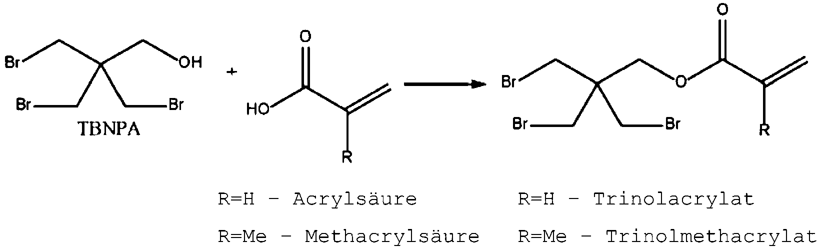 bei einem molaren Überschuss der Säure, um eine Reaktionsmischung zu bilden, die Trinol(meth)acrylat und restliches TBNPA umfasst; Zugeben eines gegenüber einem Alkohol reaktiven Abfangmittels zu der Reaktionsmischung, um restliches TBNPA zu eliminieren und im Wesentlichen TBNPA-freies Trinol(meth)acrylat zu gewinnen.

12. Prozess nach Anspruch 11, wobei die Reaktion von (Meth)acrylsäure und TBNPA in einem organischen Lösungsmittel in Gegenwart eines Veresterungskatalysators und eines Polymerisationsinhibitors bei Rückflusstemperatur stattfindet, gebildetes Wasser durch azeotrope Destillation entfernt wird, während das organische Lösungsmittel kondensiert und in das Reaktionsgefäß zurückgeführt wird.

13. Prozess nach Anspruch 11, wobei das zugesetzte Abfangmittel ein Veresterungsmittel ist, dessen Reaktivität gegenüber einem Alkohol stärker ist als die von (Meth)acrylsäure, wobei das Abfangmittel aus reaktiven Derivaten von (Meth)acrylsäure ausgewählt wird.

14. Prozess nach Anspruch 12, wobei das Veresterungsmittel (Meth)acrylsäureanhydrid oder Acryloylchlorid ist:

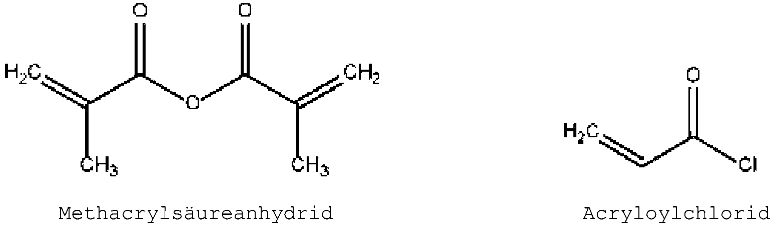

15. Prozess nach Anspruch 14, wobei die Veresterungsreaktion zur Entfernung von TBNPA unter Erhitzen stattfindet, wobei Methacrylsäureanhydrid in Gegenwart einer stickstoffhaltigen organischen Basis verwendet wird, um die Veresterungsreaktion von TBNPA und Methacrylsäureanhydrid zu katalysieren.

## Revendications

1. Composition comprenant un polyester insaturé dissous dans une solution de monomère avec du (méth)acrylate de trinol, dans laquelle la composition est essentiellement exempte d'alcool tribromonéopentylique (TBNPA) de formule :

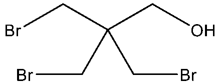

2. Composition selon la revendication 1, comprenant de 25 à 45 parties d'acrylate de trinol pour cent parties de résine (phr), dans laquelle l'acrylate de trinol présente la formule :

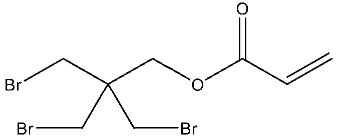

3. Composition selon la revendication 1 ou 2, dans laquelle la concentration de TBNPA est inférieure à 0,3 % en poids, calculée sur la base du poids total de la composition.

4. Composition selon les revendications précédentes, comprenant un accélérateur.

5. Composition selon la revendication 4, comprenant un initiateur de radicaux libres.

6. Procédé d'amélioration de la résistance au feu d'un polyester réticulé, comprenant l'ajout d'un (méth)acrylate de trinol essentiellement exempt de TBNPA, d'un accélérateur et d'un initiateur de radicaux libres à une solution de polyester insaturé dans un solvant monomère, formant ainsi une composition durcissable et l'initiation de la réticulation, pour réticuler le polyester.

7. Procédé selon la revendication 6, dans lequel la concentration de TBNPA dans la composition durcissable est inférieure à 0,3 % en poids, sur la base du poids total de la composition.

8. Polyester réticulé pouvant être obtenu par les procédés selon les revendications 6 ou 7.

9. Polyester réticulé au (méth)acrylate de trinol, contenant moins de 0,3 % en poids de TBNPA, sur la base du poids total du polyester réticulé.

10. Polyester réticulé au (méth)acrylate de trinol selon la revendication 9, dans lequel la teneur en brome du polyester réticulé est de 14 à 18 pour cent en poids, sans atomes de brome le long de la chaîne principale du polyester, les atomes de brome étant exclusivement incorporés dans l'unité structurale correspondant au (méth)acrylate de trinol qui relie deux chaînes de polyester, dans lequel la concentration de TBNPA non copolymérisé est inférieure à 0,1 % en poids, calculée sur la base du poids total du polyester réticulé.

11. Processus de préparation d'un (méth)acrylate de trinol essentiellement exempt de TBNPA, comprenant la réaction de l'acide (méth)acrylique avec le TBNPA : 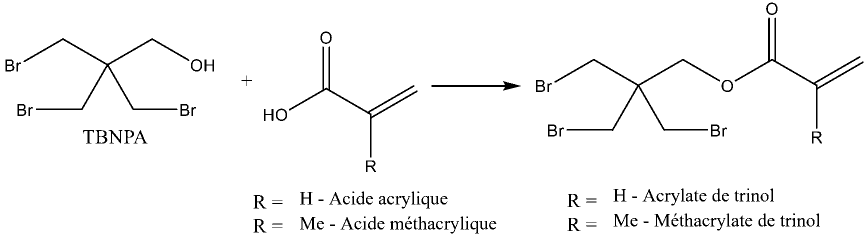 avec un excès molaire de l'acide, pour former un mélange réactionnel comprenant du (méth)acrylate de trinol et du TBNPA résiduel ; l'ajout au mélange réactionnel d'un agent piégeur réactif vis-à-vis d'un alcool pour éliminer le TBNPA résiduel, puis la récupération d'un (méth)acrylate de trinol essentiellement exempt de TBNPA.

12. Processus selon la revendication 11, dans lequel la réaction de l'acide (méth)acrylique et du TBNPA a lieu dans un solvant organique en présence d'un catalyseur d'estérification et d'un inhibiteur de polymérisation à température de reflux, l'eau formée est éliminée par distillation azéotropique tandis que le solvant organique est condensé et renvoyé dans le récipient de réaction.

13. Processus selon la revendication 11, dans lequel l'agent piégeur ajouté est un agent d'estérification dont la réactivité vis-à-vis d'un alcool est supérieure à celle de l'acide (méth)acrylique, l'agent piégeur étant choisi parmi des dérivés réactifs de l'acide (méth)acrylique.

14. Processus selon la revendication 12, dans lequel l'agent d'estérification est l'anhydride (méth)acrylique ou le chlorure d'acryloyle :

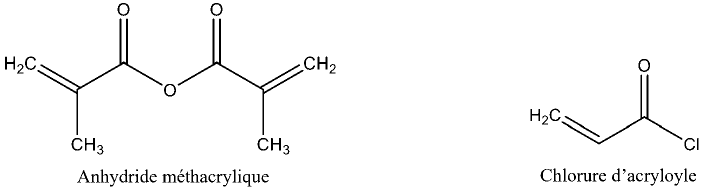

15. Processus selon la revendication 14, dans lequel la réaction d'estérification destinée à éliminer le TBNPA a lieu sous chauffage, en utilisant de l'anhydride méthacrylique en présence d'une base organique azotée pour catalyser la réaction d'estérification entre le TBNPA et l'anhydride méthacrylique.
